# EUROPEAN PATENT SPECIFICATION

(11) **EP 4 327 813 B1**
(45) Date of publication and mention of the grant of the patent: **08.04.2026**
(21) Application number: 22792076.6
(22) Date of filing: 22.04.2022
(51) Int. Cl.: A61K 31/519, A61K 31/4738, A61K 31/53, A61K 31/4985, A61K 31/522, A61K 31/506, A61K 31/573, A61K 31/567, A61K 31/4745, A61K 31/58, A61P 25/28

(54) **COADMINISTRATION OF MIRODENAFIL WITH CORT-108297 FOR PREVENTING AND TREATING DEMENTIA**
VERABREICHUNG VON MIRODENAFIL MIT CORT-108297 ZUR PRÄVENTION UND BEHANDLUNG VON DEMENZ
COADMINISTRATION DU MIRODÉNAFIL AVEC CORT-108297 POUR LA PRÉVENTION ET LE TRAITEMENT DE LA DÉMENCE

(30) Priority: 23.04.2021 KR 20210052930
(43) Date of publication of application: 28.02.2024
(73) Proprietor: ARIBIO CO., LTD., Seongnam-si, Gyeonggi-do 13535 (KR)
(72) Inventor: CHOUNG, Jaijun, Seoul 06194 (KR); KANG, Byungwoo, Daegu 42509 (KR)
(74) Representative: V.O.
(86) International application number: PCT/KR2022/005818
(87) International publication number: WO 2022/225378

(56) References cited:
- WO-A1-2014/088326
- WO-A2-2020/201915
- KR-A- 20110 010 743
- KR-A- 20200 010 224
- KR-B1- 102 000 382
- KR-B1- 102 311 224
- PINEAU FANNY ET AL: "New selective glucocorticoid receptor modulators reverse amyloid-[beta] peptide-induced hippocampus toxicity", NEUROBIOLOGY OF AGING, TARRYTOWN, NY, US, vol. 45, 27 May 2016 (2016-05-27), pages 109 - 122, XP029671648, ISSN: 0197-4580, DOI: 10.1016/J.NEUROBIOLAGING.2016.05.018
- SOLOMON MATIA B., WULSIN AYNARA C., RICE TAYLOR, WICK DAYNA, MYERS BRENT, MCKLVEEN JESSICA, FLAK JONATHAN N., ULRICH-LAI YVONNE, H: "The selective glucocorticoid receptor antagonist CORT 108297 decreases neuroendocrine stress responses and immobility in the forced swim test", HORMONES AND BEHAVIOR, ACADEMIC PRESS, NEW YORK, NY, US, vol. 65, no. 4, 1 April 2014 (2014-04-01), US , pages 363 - 371, XP055979453, ISSN: 0018-506X, DOI: 10.1016/j.yhbeh.2014.02.002

## Description

### FIELD OF THE INVENTION

The present invention relates to the treatment of Alzheimer's disease and dementia through the removal of amyloid-beta by co-administration of mirodenafil, one of phosphodiesterase 5 inhibitors (PDES inhibitors), and CORT-108297, one of glucocorticoid receptor (GR) antagonists.

### BACKGROUND DESCRIPTION

Dementia is an acquired brain disease with multifaceted pathogenesis caused by various genetic and environmental risk factors, and refers to a clinical disease that suffers from multiple cognitive deficits. The most common cause of dementia is Alzheimer's disease, which mainly occurs in the elderly and is reported to account for about 60-70% of all dementia cases (Ann Neurol. 1993 May;33(5):494-501.).

Amyloid beta protein (Aβ), which is known to be a common cause of hereditary and sporadic Alzheimer's disease, is actively being studied. Aβ is produced in small amounts throughout the human body, even in normal people. In normal people, Aβ is quickly decomposed after it is made and does not accumulate in the human body, but in patients with Alzheimer's disease, Aβ is produced abnormally in large quantity and does not decompose and accumulates in tissues, resulting in the formation of senile plaques or excessive accumulation in places such as the hippocampus and cerebral cortex, which play an important role in memory and learning. The accumulated Aβ causes an inflammatory response in the surrounding cells. As a result, nerve cells are damaged and, gradually, the neural circuitry that maintains the normal functioning of the brain is compromised. In addition, the accumulated Aβ produces a lot of free radicals that activate the signaling system that kills nerve cells.

Aβ is part of an amyloid precursor protein cleaved by β-secretase. Aβ comes in several forms, depending on the number of amino acids that make it up. In patients with Alzheimer's disease, the ratio of Aβ, which is made up of 40 or 42 amino acids, increases dramatically. When Aβ is treated with nerve cells cultured in vitro, it induces neuronal death, and there are many reports that the mechanism of cell death is similar to the type of apoptosis seen in patients with Alzheimer's disease . Damage to nerve cells by Aβ1-42 or Aβ1-43 proteins has been identified as one of the important causes of Alzheimer's disease (Yan, S, D, et al. Nature 1997 Oct 16;389(6652):689-95.) (Haass C, Selkoe DJ. Cell 1993 Dec 17;75(6):1039-42.), Aβ25-35 is known to be an important toxic fragment of Aβ1-42 or 43 that causes damage to nerve cells. Pike CJ, Ramezan-Arab N, Cotman CW. J Neurochem 1997 Oct;69(4):1601-11.) (Lorenzo A, Yankner BA. Ann NY Acad Sci 777, 89-95, 1996.)

Some of the most commonly FDA-approved drugs currently used to treat dementia include AChEl and N-Methyl-D-aspartate receptor antagonists, as well as antioxidants, nonsteroidal anti-inflammatory drugs (NSAIDs), anti-inflammatory agents, statins, and hormones. Various drugs such as preparations are used interchangeably with them. However, these drugs are only used to alleviate symptoms, delay and improve cognitive function, but no fundamental treatment for dementia has been developed.

Typical AChEls include donepezil, rivastigmine, and galantamine, which have a symptomatic treatment effect by temporarily increasing the concentration of the neurotransmitter acetylcholine. These drugs are also prescribed for mild to moderate Alzheimer's disease, vascular dementia, Parkinson's disease dementia and stroke or subcortical ischemic vascular disease (J Korean Med Assoc 2009; 52(4): 417-425.).

Currently, global pharmaceutical companies are trying to develop drugs that inhibit the action of β-secretase to block the production of Aβ at the source. In recent years, pharmaceutical companies, universities, and research institutes in the United States have been conducting many studies to isolate components that exhibit antioxidant effects from medicinal plants for the development of dementia treatments, and among them, the ingredients that have begun to be used as a treatment for dementia are Ginkgo biloba L (Ginkgo biloba) (Le Bars, P L et al. JAMA. 1997 Oct 22-29;278(16):1327-32.) (Oken, B S et al. Arch Neurol. 1998 Nov;55(11):1409-15.)and Huperzia serrata Travis (Chinese moss) (Badia, A et al. Bioorg Med Chem. 1998 Apr;6(4):427-40.) (Skolnick, A A. JAMA. 1997 Mar 12;277(10):776.)These are typical cases. In addition, curcuminoids isolated from Curcuma longa L. (turmeric) have also been reported to have therapeutic efficacy in dementia (Bastianetto, S et al. Eur J Neurosci. 2000 Jun;12(6):1882-90)(Zangara, Andrea. Pharmacol Biochem Behav. 2003 Jun;75(3):675-86.) (Park SY, Kim DS. J Nat Prod. 2002 Sep;65(9):1227-31) Ginseng, tetrandrine, and ginkgo biloba extract (EGB761), which are known to inhibit the toxicity of amyloid metabolites, have been reported to increase neuronal survival or slow the rate of worsening of dementia symptoms in Alzheimer's patients. Bastianetto, S et al. Eur J Neurosci. 2000 Jun;12(6):1882-90)

On the other hand, there is no satisfactory treatment method for Alzheimer's dementia, and effective anti-dementia drugs have not yet been developed.

### DETAILED DESCRIPTION OF THE INVENTION

### Technical challenges

Degenerative neurological diseases, including dementia, are caused by the reduction or loss of nerve cell function, which causes abnormalities in a wide variety of functions, including autonomic nervous function, which is self-regulating without perceiving as well as all functions of the body that we can feel, such as motor control function, cognitive function, perceptive function, and sensory function.

To date, the cause of dementia has not been identified, so fundamental treatment is not possible, and the five commercialized drugs can only alleviate symptoms in some diseases, and these drugs have only the effect of symptom completion that does not fundamentally change the progression of dementia.

The present invention provides a method for treating dementia by protecting nerve cells by reducing amyloid beta by using mirodenafil in combination with CORT-108297, which is being developed as a treatment for post-traumatic stress disorder (PTDS), against amyloid beta, which is a direct cause of neuronal death.

### Means of solving the problem

The present invention is a phosphodiesterase 5 inhibitor; and glucocorticoid receptor (GR) antagonists. It relates to a composition for the prevention and treatment of dementia containing as an active ingredient.

In the present invention, dementia includes Alzheimer's dementia, AIDS-induced dementia, Lewy body dementia, frontotemporal dementia, multiple infarction dementia, semantic dementia and vascular dementia, and Huntington's disease, Parkinson's disease, and amyotrophic lateral sclerosis.

The phosphodiesterase 5 inhibitor of the present invention comprises mirodenafil and at least one species selected from a group consisting of pharmaceutically acceptable salts, solvates and hydrates thereof.

The pharmaceutically acceptable salt means a formulation of a compound that does not cause serious irritation to the organism to which the compound is administered and does not impair the biological activity and physical properties of the compound. The pharmaceutically acceptable salts are prepared by conventional methods well known in the art using pharmaceutically acceptable organic and inorganic acids that are substantially non-toxic. The acids are inorganic acids such as hydrochloric acid, bromic acid, sulfuric acid, nitric acid, phosphoric acid, methanesulfonic acid, ethanesulfonic acid, sulfonic acid such as p-toluenesulfonic acid, tataric acid, formic acid, citric acid, acetic acid, trichloroacetic acid, trifluoroacetic acid, capric acid, isobutanoic acid, It includes organic acids such as malonic acid, succinic acid, phthalic acid, gluconic acid, benzoic acid, lactic acid, fumaric acid, maleic acid, salicylic acid, and the like. In addition, by reacting the compounds of the present invention with bases, ammonium salts; alkali metal salts, such as sodium or potassium salts; salts such as calcium or magnesium salts, alkali earth metal salts, etc.; Salts of organic bases such as dicyclohexylamine, N-methyl-D-glucamine, tris (hydroxymethyl) methylamine and the like; And amino acid salts such as arginine and lysine may be formed.

According to an embodiment of the present invention, the pharmaceutically acceptable salts may illustrate mirodenafil hydrochloride

The hydrate refers to a compound of the present invention or a salt thereof comprising a stoichiometric or non-sto ichiometric amount of water bonded by a non-covalent intermo lecular force.

The solvate means a compound of the present invention or a salt thereof comprising a stoichiometric or non-stoichiometric amount of solvent bonded by a non-covalent intermolecular force. Preferred solvents are volatile, non-toxic, and / or solvents suitable for administration to humans.

Glucocorticoid Receptor (GR) Antagonist), CORT-108297, and at least one species selected from a group consisting of pharmaceutically acceptable salts, solvates and hydrates thereof.

The phosphodiesterase 5 inhibitor of the present invention is selected from the group consisting of mirodenafil and its pharmaceutically acceptable salts, solvates and hydrates, and the glucocorticoid receptor antagonist (GR) Antagonist is consisting of CORT-108297 and pharmaceutically acceptable salts, solvates and hydrates thereof.

CORT-108297 of the present invention is (R)-(4a-ethoxymethyl-1-(4-fluorophenyl)-6-(4-trifluoromethyl-benzenesulfonyl)-4,4a,5,6,7,8-hexahydro-1H,1,2,6-triaza-cyclopenta[b] naphthalene, which has the following structure:

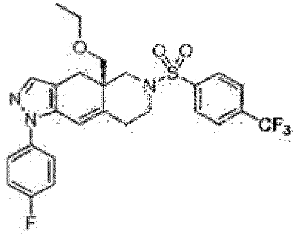

The pharmaceutical composition of the present invention can be administered orally or parenterally.

According to an embodiment of the present invention, the pharmaceutical composition of the present invention is orally administered to a subject, or non-orally administered to a site other than the head. That is, the compositions of the present invention may exhibit the intended effect in the present invention even when not directly administered to brain tissue, body tissues surrounding brain tissue (eg, scalp) and adjacent areas. In one particular example, the non-oral administration is subcutaneous administration, intravenous administration, abdominal infusion, transdermal administration, or intramuscular administration, and in another particular example, subcutaneous administration, intravenous administration , or intramuscular administration.

The pharmaceutically acceptable carriers contained in the pharmaceutical composition of the present invention are commonly used in the formulation of lactose, dextrose, sucrose, sorbitol, mannitol, starch, acacia rubber, calcium phosphate, alginate, gelatin, calcium silicate, microcrystalline cellulose, polyvinylpyrrolidone , cellulose, water , Syrup, methyl cellulose, methyl hydroxybenzoate, propyl hydroxybenzoate, talc, magnesium stearate and mineral oil , but are not limited to. The pharmaceutical composition of the present invention may additionally comprise a lubricant, a humectant, a sweetener, a flavoring agent, an emulsifier, a suspension, a preservative , and the like in addition to the above components. Suitable pharmacologically acceptable carriers and formulations are described in detail in Remington's Pharmaceutical Sciences (19th ed., 1995).

The pharmaceutical composition of the present invention may be prepared in a unit dose form or in a multi-capacity container by formulation using a pharmaceutically acceptable carrier and / or excipient according to a method that can be easily performed by a person skilled in the art to which the invention belongs. In this case, the formulation may be in the form of a solution, suspension or emulsion in an oil or aqueous medium, or in the form of an X-agent, powder, granule, tablet, film or capsule, and may additionally include a dispersant or stabilizer.

### Effect of the invention

The present invention relates to the effect of inhibiting dementia through amyloid beta reduction by combining phosphodiesterase 5 inhibitor and glucocorticoid receptor antagonist.

### A BRIEF DESCRIPTION OF THE DRAWING

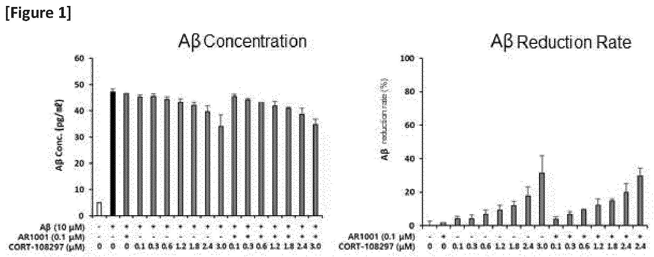

FIG. 1 is an experimental result of a reduction in intracellular Aβ by co-treatment of mirodenafil (AR1001) and CORT-108297 according to an embodiment of the present invention.

### EMBODIMENT OF THE INVENTION

Hereinafter, the present invention will be described in more detail according to the following embodiments. However, these embodiments are only for illustrative purposes of the present invention, and the scope of the present invention is not limited by these embodiments.

### [Experiment Example 11 Cell culture

The SH-SY5Y human neuroblastoma cell line used in the experiment was obtained from the American Type Culture Collection (ATCC; Manassas, VA, USA) and 10% fetal bov ine serum(FBS; Australian Orgin, HyClone, Logan, UT, USA) and 1% penicillin/streptom ycin (P/S; HyClone) was incubated in a CO2 incubator (311-TIF, Thermo Fisher Scientific Forma, MA, USA) under 37 ° C. and 5% CO2 conditions using DMEM / F12 Complete Medium (HyClone).

### [Experimental Example 2] neuron-like differentiationof SH-SY5Y cells using all-trans-retinoic acid (RA)

To determine the amount of change in amyloid beta, 2*?*⁵ cells were dispensed into T-25 flask.

For cell adhesion and stabilization, cells were incubated for 24 hours in DMEM /F12 Complete Medium (HyClone) containing 10% FBS (HyClone) and 1% P/S (HyClone) at 37 °C under 5% CO₂ condition in a CO₂ incubator (Thermo Fisher Scientific Forma).

After 24 hours of cell dispensing, the cell culture medium is removed for neuron-like differentiation, and replaced with DMEM/F12 differentiation media containing 1% FBS (HyClone), 1% P/S (HyClone), 10 µM all-trans-retinoic acid (RA; Sigma-Aldrich, St. Louis, MO, USA).

On the third day of differentiation, the medium was replaced with a new DMEM/F12 differentiation medium. On the sixth day of differentiation, the medium of the untreated control group was replaced with a new DMEM/F12 differentiation medium and the medium of the sample treatment group was replaced with a various conditions by adding a new DMEM/F12 differentiation medium under various conditions.

### [Experimental Example 3] Amyloid β(Aβ)1-42 Formation and treatment

Human Aβ1-42 (Abcam, Cambridge, MA, USA) was added upto 10 µM to DMEM/F12 Complete Medium (HyClone) containing 1% FBS (HyClone) and 1% P/S (HyClone) to form Aβ1-42 oligomer and left for 3 hours in a CO₂ incubator (Thermo Fisher Scientific Forma) under 37 °C. and 5% CO₂ conditions to form Aβ1-42 oligomer.

In order to confirm the Aβ 1-42 change, the existing cell culture medium was removed from the RA-differentiated SH-SY5Y neuron-like cells, replaced with DMEM/F12 Complete Medium (HyClone) containing Aβ1-42 oligomer (10 µM) and incubated for 72 hours in a CO₂ incubator (Thermo Fisher Scientific Forma) at 37 °C under 5% CO₂ conditions to induce Aβ1-42 oligomer-induced cell damage.

After 72 hours, the medium was removed and the cells were treated with of DME M/F12 Complete Medium (HyClone) containing 10 µM of Aβ1-42 oligomer alone or in combination with mirodenafil and CORT-108297 and incubated in a CO₂ incubator (Thermo Fisher Scientific Forma) at 37 °C under 5% CO₂ for 24 hours before the next experiments.

### [Example 4] Amyloid beta 42 Human ELISA (enzyme-linked immunosorbent assay) Measurement results

In order to measure the amount of Aβ42 (pg/mL) in the cell, the cells were recovered and treated with the cell lysis buffer.

Thereafter, after centrifugation at 14,000 rpm 4 °C for 10 minutes, the supernatant was transferred to recover the protein. The amount of protein was quantified using the Pierce^{™} BCA Protein Assay Kit (Thermo Fisher Scientific). Then, the amount of Aβ42 in cells was measured using a Human Aβ42 ELISA Kit (Invitrogen).

**[Table 1]**

| Sample | | | Aβ42 concentration | | Aβ42 Reduction rate | | Combination Index | |
|---|---|---|---|---|---|---|---|---|
| AR100 01 | CORT-10829 7 (µM) | Aβ (10µ M) | Average concentrati on | Concentrati on Stdev | Averag e reducti on rate | Reducti on rate | Group | Valu e |
| - | 0 | - | 4.99 | 0.19 | - | | - | |
| - | 0 | + | 47.19 | 1.16 | 0.00 | 2.75 | - | |
| + | 0 | + | 46.45 | 0.11 | 1.77 | 0.25 | A | |
| - | 0.1 | + | 45.40 | 0.59 | 4.26 | 1.39 | B | |
| - | 0.3 | + | 45.40 | 0.95 | 4.24 | 2.24 | | |
| - | 0.6 | + | 44.29 | 1.05 | 6.88 | 2.49 | | |
| - | 1.2 | + | 43.25 | 1.26 | 9.34 | 2.98 | | |
| - | 1.8 | + | 42.14 | 1.15 | 11.97 | 2.73 | | |
| - | 2.4 | + | 39.64 | 2.19 | 17.91 | 5.18 | | |
| - | 3.0 | + | 33.97 | 4.47 | 31.34 | 10.59 | | |
| + | 0.1 | + | 45.59 | 0.66 | 3.81 | 1.56 | Combinati on | 0.63 |
| + | 0.3 | + | 44.22 | 0.52 | 7.06 | 1.24 | | 1.17 |
| + | 0.6 | + | 43.18 | 0.10 | 9.52 | 0.25 | | 1.10 |
| + | 1.2 | + | 41.99 | 1.57 | 12.32 | 3.72 | | 1.11 |
| + | 1.8 | + | 40.89 | 0.42 | 14.95 | 0.99 | | 1.09 |
| + | 2.4 | + | 38.75 | 2.18 | 20.00 | 5.17 | | 1.02 |
| + | 3.0 | + | 34.72 | 2.07 | 29.57 | 4.91 | | 0.89 |

In conclusion, the Aβ reduction rate was 7.06% in mirodenafil 0.1 µM and CORT-108297 0.3 µM combo treatment group; 9.52% Aβ reduction rate in mirodenafil 0.1 µM and CORT-108297 0.6 µM combo treatment group; 12.32% Aβ reduction rate in mirodenafil 0.1 µM and CORT-108297 1.2 µM combo treatment group; 14.95% Aβ reduction rate in mirodenafil 0.1 µM and CORT-108297 1.8 µM combo treatment group; 20.00% Aβ reduction rate in mirodenafil 0.1 µM and CORT-108297 2.4 µM combo treatment group; all of which were higher than the sum of A and B reduction rate from treatment with mirodenafil or CORT-108297 individually and thus, it was confirmed that the effect was more than additive and synergistic.

The present invention described above is only an example, and a person of ordinary skill in the art to which the present invention belongs will be well aware that various modifications and uniform other embodiments are possible therefrom. Therefore, it may be well understood that the present invention is not limited to the forms referred to in the above detailed description. Therefore, the true scope of technical protection of the present invention should be determined by the technical ideas of the attached claims. In addition, the present invention should be understood to include the spirit of the present invention as defined by the attached claims and all modifications, equals, and substitutes within the scope thereof.

## Claims

1. A composition for use in the prevention or treatment of dementia comprising:
a phosphodiesterase 5 inhibitor; and
a glucocorticoid receptor antagonist as the active ingredient,
wherein,
the phosphodiesterase 5 inhibitor is at least one selected from the group consisting of mirodenafil and a pharmaceutically acceptable salt, solvate or hydrate thereof; and
the glucocorticoid receptor antagonist is selected from the group consisting of CORT-108297 and its pharmaceutically acceptable salts, solvates or hydrates;
wherein said prevention or treatment comprises co-administration of the at least one of the phosphodiesterase 5 inhibitors and the glucocorticoid receptor antagonists at a molar concentration ratio of 1:3 - 1:24 to have a synergistic effect on the removal of amyloid beta (AB).

2. A composition for use according to claim 1, wherein use in the prevention or treatment of dementia includes use in the prevention or treatment of Alzheimer's dementia, AIDS-induced dementia, Lewy body dementia, frontotemporal dementia, multiple infarction dementia, semantic dementia, vascular dementia, Huntington's disease, Parkinson's disease, and amyotrophic lateral sclerosis.

3. A composition for use according to claim 1 or 2, wherein the composition further comprises a pharmaceutically acceptable carrier selected from the group of lactose, dextrose, sucrose, sorbitol, mannitol, starch, acacia rubber, calcium phosphate, alginate, gelatin, calcium silicate, microcrystalline cellulose, polyvinylpyrrolidone, cellulose, water, syrup, methyl cellulose, methyl hydroxybenzoate, propyl hydroxybenzoate, talc, magnesium stearate and mineral oil.

4. A composition for use according to claim 3, wherein the composition further optionally comprises a lubricant, a humectant, a sweetener, a flavoring agent, an emulsifier, a suspension, or a preservative.

5. A composition for use according to any of claims 1 - 4, wherein said use comprises oral administration or parenteral administration.

6. A composition for use according to any of claims 1 - 5, wherein said use comprises subcutaneous administration, intravenous administration, abdominal infusion, transdermal administration or intramuscular administration.

## Patentansprüche

1. Zusammensetzung zur Verwendung bei der Prävention oder Behandlung von Demenz, umfassend:
einen Phosphodiesterase-5-Inhibitor; und
einen Glukokortikoidrezeptorantagonisten als Wirkstoff,
wobei
der Phosphodiesterase-5-Inhibitor mindestens einer ist, ausgewählt aus der Gruppe bestehend aus Mirodenafil und einem pharmazeutisch akzeptablen Salz, Solvat oder Hydrat davon; und
der Glukokortikoid-Rezeptorantagonist ausgewählt ist aus der Gruppe bestehend aus CORT-108297 und seinen pharmazeutisch akzeptablen Salzen, Solvaten oder Hydraten;
wobei die Prävention oder Behandlung die Ko-Verabreichung des wenigstens einen von den Phosphodiesterase-5-Inhibitoren und der Glukokortikoidrezeptorantagonisten in einem molaren Konzentrationsverhältnis von 1:3 bis 1:24 umfasst, um eine synergistische Wirkung auf die Entfernung von Amyloid-Beta (AB) zu haben.

2. Zusammensetzung zur Verwendung nach Anspruch 1, wobei die Verwendung zur Prävention oder Behandlung von Demenz die Verwendung zur Prävention oder Behandlung von Alzheimer-Demenz, AIDS-induzierter Demenz, Lewy-Körper-Demenz, frontotemporaler Demenz, Multi-Infarkt-Demenz, semantischer Demenz, vaskulärer Demenz, Huntington-Krankheit, Parkinson-Krankheit und amyotropher Lateralsklerose einschließt.

3. Zusammensetzung zur Verwendung nach Anspruch 1 oder 2, wobei die Zusammensetzung ferner einen pharmazeutisch akzeptablen Träger umfasst, ausgewählt aus der Gruppe von Lactose, Dextrose, Saccharose, Sorbitol, Mannit, Stärke, Akaziengummi, Calciumphosphat, Alginat, Gelatine, Calciumsilikat, mikrokristalliner Cellulose, Polyvinylpyrrolidon, Cellulose, Wasser, Sirup, Methylcellulose, Methylhydroxybenzoat, Propylhydroxybezoat, Talkum, Magnesiumstearat und Mineralöl .

4. Zusammensetzung zur Verwendung nach Anspruch 3, wobei die Zusammensetzung ferner optional ein Schmiermittel, einen Feuchthaltemittel, ein Süßungsmittel, einen Aromastoff, einen Emulgator, eine Suspension oder ein Konservierungsmittel umfasst.

5. Zusammensetzung zur Verwendung nach einem der Ansprüche 1 bis 4, wobei die Verwendung orale Verabreichung oder die parenterale Verabreichung umfasst.

6. Zusammensetzung zur Verwendung nach einem der Ansprüche 1 bis 5, wobei die Verwendung subkutane Verabreichung, intravenöse Verabreichung, abdominale Infusion, transdermale Verabreichung oder intramuskuläre Verabreichung umfasst.

## Revendications

1. Composition pour une utilisation dans la prévention ou le traitement d'une démence, comprenant :
un inhibiteur de phosphodiestérase 5 ; et
un antagoniste de récepteur de glucocorticoïde en tant que principe actif,
dans laquelle
l'inhibiteur de phosphodiestérase 5 est au moins l'un choisi dans le groupe constitué par le mirodénafil et un sel, solvate ou hydrate pharmaceutiquement acceptable de celui-ci ; et
l'antagoniste de récepteur de glucocorticoïde est choisi dans le groupe constitué par le CORT-108297 et ses sels, solvates et hydrates pharmaceutiquement acceptables ;
dans laquelle ladite prévention ou ledit traitement comprend la coadministration de l'au moins un des inhibiteurs de phosphodiestérase 5 et des antagonistes de récepteur de glucocorticoïde à un rapport de concentration molaire de 1/3 à 1/24 pour qu'il y ait un effet synergique sur l'élimination d'amyloïde bêta (Aβ).

2. Composition pour une utilisation selon la revendication 1, dans laquelle une utilisation dans la prévention ou le traitement d'une démence comprend une utilisation dans la prévention ou le traitement d'une démence d'Alzheimer, d'une démence induite par le SIDA, d'une démence à corps de Lewy, d'une démence fronto-temporale, d'une démence multi-infarctus, d'une démence sémantique, d'une démence vasculaire, d'une chorée de Huntington, d'une maladie de Parkinson, et d'une sclérose latérale amyotrophique.

3. Composition pour une utilisation selon la revendication 1 ou 2, dans laquelle la composition comprend en outre un véhicule pharmaceutiquement acceptable choisi dans le groupe comprenant le lactose, le dextrose, le saccharose, le sorbitol, le mannitol, l'amidon, la gomme arabique, le phosphate de calcium, l'alginate, la gélatine, le silicate de calcium, la cellulose microcristalline, la polyvinylpyrrolidone, la cellulose, l'eau, le sirop, la méthylcellulose, l'hydroxybenzoate de méthyle, l'hydroxybenzoate de propyle, le talc, le stéarate de magnésium et l'huile minérale.

4. Composition pour une utilisation selon la revendication 3, dans laquelle la composition comprend éventuellement en outre un lubrifiant, un humectant, un édulcorant, un agent aromatisant, un émulsifiant, une suspension, ou un conservateur.

5. Composition pour une utilisation selon l'une quelconque des revendications 1 à 4, dans laquelle ladite utilisation comprend une administration par voie orale ou une administration par voie parentérale.

6. Composition pour une utilisation selon l'une quelconque des revendications 1 à 5, dans laquelle ladite utilisation comprend une administration par voie sous-cutanée, une administration par voie intraveineuse, une administration par voie abdominale, une administration par voie transdermique ou une administration par voie intramusculaire.
